(19) **Europäisches Patentamt**
**European Patent Office**
**Office européen des brevets**

(11) **EP 2 686 261 B1**

(12) **EUROPÄISCHE PATENTSCHRIFT**

(45) Veröffentlichungstag und Bekanntmachung des Hinweises auf die Patenterteilung:
**18.05.2016 Patentblatt 2016/20**

(21) Anmeldenummer: **12715566.1**

(22) Anmeldetag: **14.03.2012**

(51) Int Cl.:
***B65H 63/06*** *(2006.01)*      ***D01H 13/22*** *(2006.01)*
***G01N 33/36*** *(2006.01)*      ***G06F 17/21*** *(2006.01)*

(86) Internationale Anmeldenummer:
**PCT/CH2012/000059**

(87) Internationale Veröffentlichungsnummer:
**WO 2012/122663 (20.09.2012 Gazette 2012/38)**

(54) **CHARAKTERISIERUNG EINES LÄNGLICHEN TEXTILEN PRÜFGUTES**

CHARACTERIZING AN ELONGATED TEXTILE TEST MATERIAL

CARACTÉRISATION D'UN ÉCHANTILLON TEXTILE DE FORME ALLONGÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.03.2011 CH 460112011**

(43) Veröffentlichungstag der Anmeldung:
**22.01.2014 Patentblatt 2014/04**

(73) Patentinhaber: **Uster Technologies AG**
**8610 Uster (CH)**

(72) Erfinder:
• **SCHMID, Peter**
  **CH-8050 Zürich (CH)**
• **KELLER, Beat**
  **CH-8046 Zürich (CH)**

• **GEHRIG, Stefan**
  **CH-8610 Uster (CH)**
• **STORZ, Rafael**
  **CH-8280 Kreuzlingen (CH)**
• **CARRARO, Flavio**
  **CH-8493 Saland (CH)**

(74) Vertreter: **Pliska, Pavel**
**Uster Technologies AG**
**Sonnenbergstrasse 10**
**8610 Uster (CH)**

(56) Entgegenhaltungen:
**EP-A1- 0 685 580      EP-A2- 0 439 767**
**WO-A1-2010/078665      DE-A1- 10 348 741**
**DE-A1-102007 028 651      US-B1- 6 374 152**

• **'Anwendungshandbuch Uster Quantum 2', 01 Mai 2005, USTER Seite 4.9, XP055208431**

EP 2 686 261 B1

**Beschreibung**

Die vorliegende Erfindung liegt auf dem Gebiet der textilen Qualitätskontrolle. Sie betrifft ein Verfahren und eine Vorrichtung zur Charakterisierung eines länglichen textilen Prüfgutes, gemäss den Oberbegriffen der unabhängigen Patentansprüche. Derartige Verfahren und Vorrichtungen kommen typischerweise auf Spinn- oder Spulmaschinen zum Einsatz. Das längliche textile Prüfgut ist vorzugsweise ein Garn, kann aber auch ein Faserband oder ein Vorgarn etc. sein.

Die Erfindung bezieht sich auch auf eine Textilverarbeitungsmaschine.

STAND DER TECHNIK

Zur Sicherung der Garnqualität werden an Spinn- oder Spulmaschinen so genannte Garnreiniger eingesetzt. Eine derartige Vorrichtung ist z. B. aus der EP-0'439'767 A2 bekannt. Sie beinhaltet einen Messkopf mit mindestens einem Sensor, der das bewegte Garn abtastet. Häufig verwendete Sensorprinzipien sind das kapazitive (siehe z. B. EP-0'924'513 A1) oder das optische (siehe z. B. WO-2004/044579 A1). Ziel der Abtastung ist es, Fehlstellen wie Dickstellen, Dünnstellen oder Fremdstoffe im Garn zu detektieren. Das Ausgangssignal des Sensors wird laufend mit vorgegebenen Bewertungskriterien bewertet. Die Bewertungskriterien werden üblicherweise in Form einer Reinigungsgrenze bzw. Reinigungskurve in einem zweidimensionalen Ereignisfeld vorgegeben, welches von der Länge des Ereignisses einerseits und von einer Amplitude des Ereignisses, z. B. einer Abweichung der Garnmasse von einem Sollwert, andererseits aufgespannt wird. Ereignisse unterhalb der Reinigungsgrenze werden toleriert, Ereignisse oberhalb der Reinigungsgrenze werden aus dem Garn entfernt oder zumindest als Fehlstellen registriert. Im Anwendungshandbuch "USTER® QUANTUM 2 - Online-Qualitätsmanagement in der Spulerei", Uster Technologies AG, Mai 2005, ist beschrieben, dass der Verlauf der Reinigungsgrenze mit Hilfspunkten den Garnfehlern angepasst werden kann. Die Koordinaten der Hilfspunkte werden zusammen mit der Reinigungsgrenze angezeigt.

Seit Jahrzehnten ist es üblich, Garnfehler mit dem System USTER® *CLASSIMAT* der Anmelderin des vorliegenden Schutzrechtes zu klassieren. Dieses System ist z. B. im Patent US-5,537,811 A und in der Broschüre "USTER® CLASSIMAT QUANTUM", Uster Technologies AG, August 2007, beschrieben. Demnach wird das oben erwähnte Ereignisfeld in eine diskrete Anzahl, bspw. 23, von rechteckigen Klassen unterteilt, wodurch ein Klassierfeld entsteht. Jedem Garnfehler kann dann gemäss seiner Länge und Amplitude eine Klasse zugeordnet werden. Die festgestellten Garnfehler in jeder Klasse werden gezählt und auf eine Standard-Garnlänge von bspw. 100 km umgerechnet. Die Gesamtheit der so erhaltenen Zählwerte in jeder Klasse charakterisiert das Garn und kann zur Festlegung einer Reinigungsgrenze verwendet werden. Diese Klassierung liefert einerseits relativ viele Ausgangswerte, nämlich die bspw. 23 Zählwerte; andererseits ist sie trotzdem recht grob, denn innerhalb der einzelnen Klassen werden die Ereignisse nicht mehr weiter unterschieden.

Die WO-2010/078665 A1 beschreibt ein Verfahren und eine Vorrichtung zur Charakterisierung eines entlang seiner Längsrichtung bewegten textilen Prüfgutes. Dabei werden Messwerte einer Eigenschaft des Prüfgutes entlang seiner Längsrichtung erfasst. Aus den Messwerten werden Werte eines Prüfgutparameters ermittelt. Aus den Werten des Prüfgutparameters und ihrer Erstreckung in der Längsrichtung werden Dichten von Ereignissen in dem Ereignisfeld ermittelt. In dem Ereignisfeld wird ein Prüfgutkörper als Fläche grafisch dargestellt. Die Fläche wird einerseits durch die Abszisse, andererseits durch die Ordinate und ferner durch eine Linie in dem Ereignisfeld, welche im Wesentlichen einer konstanten Ereignisdichte folgt, begrenzt. Die Darstellung des Prüfgutkörpers versetzt eine Bedienungsperson in die Lage, charakteristische Eigenschaften des Prüfgutes schnell zu erfassen und eine Reinigungsgrenze rationell vorzugeben.

DARSTELLUNG DER ERFINDUNG

Es ist eine Aufgabe der vorliegenden Erfindung, das Verfahren und die Vorrichtung gemäss der WO-2010/078665 A1 derart weiter zu entwickeln, dass die Charakterisierung des textilen Prüfgutes noch besser und objektiver wird.

Diese und andere Aufgaben werden durch das erfindungsgemässe Verfahren und die erfindungsgemässe Vorrichtung gelöst, wie sie in den unabhängigen Patentansprüchen definiert sind. Vorteilhafte Ausführungsformen sind in den abhängigen Patentansprüchen angegeben.

Die Erfindung benützt das in der WO-2010/078665 A1 eingeführte Konzept des Prüfgutkörpers, der in dem zweidimensionalen Ereignisfeld als von einer Dichtekurve begrenzte Fläche dargestellt werden kann. Die Grundidee der Erfindung ist es, dieses grafische Gebilde auf geeignete Weise numerisch fassbar zu machen. Dies kann z. B. mit Hilfe von Stützpunkten, mit einer Kurvenanpassung oder auf andere Weise geschehen.

Dementsprechend werden im erfindungsgemässen Verfahren zur Charakterisierung eines entlang seiner Längsrichtung bewegten länglichen textilen Prüfgutes Messwerte einer Eigenschaft des textilen Prüfgutes entlang der Längsrichtung des textilen Prüfgutes erfasst. Es werden Werte eines Parameters des textilen Prüfgutes aus den Messwerten ermittelt. Ein Ereignisfeld wird bereitgestellt, das einen Quadranten oder einen Teil eines Quadranten eines zweidimensionalen kartesischen Koordinatensystems beinhaltet, dessen Abszisse eine Erstreckung von Parameterwerten in der Längsrichtung und dessen Ordinate eine Abweichung des Parameters von einem Sollwert angibt. Aus den Werten des Parameters und ihrer Erstreckung in der Längsrichtung werden Dichten von Ereignissen in dem Ereignisfeld ermittelt. In dem Ereignisfeld wird ein Prüfgutkörper als Fläche berechnet, die einerseits durch die Abszisse oder eine parallel dazu verlaufende Gerade, andererseits durch die Ordinate oder eine parallel dazu verlaufende Gerade und ferner durch eine Linie in dem Ereignisfeld, welche im Wesentlichen einer konstanten Ereignisdichte folgt, begrenzt wird. Die Fläche wird numerisch spezifiziert. Mindestens ein Wert der numerischen Spezifikation wird als Charakteristikum des textilen Prüfgutes ausgegeben.

Unter dem Begriff "Fläche" wird in dieser Schrift eine Teilmenge der Ebene, die durch das zweidimensionale kartesische Koordinatensystem aufgespannt wird, verstanden. Die so definierte Fläche ist zu unterscheiden vom Flächeninhalt, der ein Mass für die Grösse der Fläche und somit eine Eigenschaft der Fläche ist. Die den Prüfgutkörper darstellende Fläche ist vorzugsweise zusammenhängend. Der Begriff "zusammenhängend" kann hier durchaus im Sinne der mathematischen Topologie verstanden werden. Dabei dürfte es in der Praxis keine Rolle spielen, ob der allgemeine Zusammenhang oder der speziellere Wegzusammenhang zur Definition verwendet wird. Die Fläche braucht aber nicht einfach zusammenhängend zu sein, d. h. sie darf allseitig umschlossene Aussparungen aufweisen.

Die numerische Spezifikation kann z. B. die folgenden Eigenschaften der Fläche berücksichtigen:

- einen Flächeninhalt der Fläche,
- eine geometrische Form der Fläche, z. B. eine Lage eines Flächenschwerpunktes oder eines Konturenschwerpunktes der Fläche, und/oder
- einen Verlauf der Linie, welche die Fläche ferner begrenzt. Dabei kann die numerische Spezifikation anhand von Stützpunkten erfolgen. Ein bestimmtes stützpunktbasiertes Spezifikationssystem wird durch die Vorgabe der Anzahl der verwendeten Stützpunkte und ihrer Lagen auf der Abszisse definiert.

Wird die genannte Linie berücksichtigt, so kann die numerische Spezifikation anhand einer Ausgleichsrechnung erfolgen. Entsprechende mathematische Methoden der Ausgleichsrechnung sind an sich bekannt. Vorzugsweise werden auf der Linie liegende Datenpunkte gewählt und mit einem Funktionsfit unter Berücksichtung der Methode der kleinsten Quadrate angenähert. Ein bestimmtes fitbasiertes Spezifikationssystem wird durch die Vorgabe der Fitfunktion definiert. Es kommen viele verschiedene Fitfunktionen in Frage, deren gemeinsames Merkmal ein Abklingen für grosse Abszissenwerte sein sollte. Die resultierenden Funktionsparameterwerte werden dann zur Charakterisierung des untersuchten Prüfgutes verwendet.

Zur Spezifikation mittels einer Linie - sei es die genannte Linie oder eine Fitkurve - kann mindestens ein Extrempunkt, mindestens ein Wendepunkt, ein Linienschwerpunkt und/oder mindestens eine Kurvensteigung berechnet werden. Auch weitere numerische Werte, wie sie etwa aus der mathematischen Kurvendiskussion bekannt sind, können angegeben werden.

Es kann vorteilhaft sein, für die numerische Spezifikation einen Vertrauensbereich, vorzugsweise in Form von Vertrauensintervallen oder einer Vertrauensfläche, auszugeben.

Die konstante Ereignisdichte, welcher der genannten Linie entspricht, sollte die folgenden Kriterien erfüllen:

- Sie ist so hoch, dass die Entfernung aller Ereignisse aus dem Prüfgut, die mit dieser oder einer tieferen Ereignisdichte vorkommen, die Produktivität zu stark beeinträchtigen würde, und
- sie ist gleichzeitig so tief, dass sie noch genügend nahe bei derjenigen Fehlerdichte liegt, die aus dem Prüfgut zu entfernen sinnvoll erscheint, z. B. zwischen zehn- und hundertmal höher.

Die Ereignisdichte wird vorzugsweise auf ein kartesisches Koordinatensystem mit doppelt logarithmisch eingeteilten Achsen bezogen. Mit Ereignisdichten befasst sich ausführlich die US-6,374,152 B1. Insbesondere für Garn geeignete Grenzereignisdichten liegen zwischen 100 und 3000 Ereignissen pro 100 km Prüfgutlänge und vorzugsweise bei 1000 Ereignissen pro 100 km Prüfgutlänge.

Der Parameter entspricht vorzugsweise im Wesentlichen einer Masse pro Längeneinheit oder einem Durchmesser des textilen Prüfgutes. Er kann sich aber alternativ auf eine Reflektivität oder eine Absorptivität des textilen Prüfgutes, auf Fremdstoffe im Prüfgut oder auf andere Eigenschaften des textilen Prüfgutes beziehen.

In dem Ereignisfeld können mehrere, z. B. zwei, verschiedene Flächen, die vorzugsweise in verschiedenen Quadranten des Ereignisfeldes liegen, numerisch spezifiziert werden. Die mehreren Flächen können einzeln numerisch spezifiziert werden. Alternativ können die mehreren Flächen zu einer einzigen Fläche zusammengelegt und die aus der Zusammenlegung hervorgegangene einzige Fläche numerisch spezifiziert werden.

Der mindestens eine Wert der numerischen Spezifikation kann in ein Qualitäts-Referenzwerk für das entsprechende Prüfgut, wie die USTER® *STATISTICS* der Anmelderin des vorliegenden Schutzrechtes, aufgenommen werden.

Zu diesem Zweck wird vorzugsweise eine für die weltweite Produktion repräsentative Menge von Proben des entsprechenden Prüfgutes desselben Typs gesammelt und gemäss der vorliegenden Erfindung charakterisiert. Im Qualitäts-Referenzwerk können für den mindestens einen Wert der numerischen Spezifikation z. B. Perzentile, bezogen auf die weltweite Produktion des entsprechenden Prüfgutes, angegeben werden, vorzugsweise in Form eines Nomogramms. Ein Qualitäts-Referenzwerk, beinhaltend Qualitätsdaten für ein längliches textiles Prüfgut, enthält dementsprechend eine Charakterisierung des textilen Prüfgutes, die nach dem oben beschriebenen erfindungsgemässen Verfahren erhalten wurde. Somit ermöglicht es die Erfindung, die Qualität des textilen Prüfgutes noch vollständiger in Bezug auf eine repräsentative Menge von Prüfgütern derselben Art, die an einem anderen Ort und/oder zu einer anderen Zeit produziert wurden, einzustufen.

Die erfindungsgemässe Vorrichtung dient zur Charakterisierung eines entlang seiner Längsrichtung bewegten länglichen textilen Prüfgutes. Sie beinhaltet einen Messkopf zur Erfassung von Messwerten einer Eigenschaft des textilen Prüfgutes entlang der Längsrichtung des textilen Prüfgutes sowie zur Ermittlung von Werten eines Parameters des textilen Prüfgutes aus den Messwerten. Ferner beinhaltet sie eine mit dem Messkopf verbundene Steuereinheit. Die Steuereinheit hat eine Speichereinheit und eine Ausgabeeinheit zur Speicherung bzw. Ausgabe eines Ereignisfeldes, das einen Quadranten oder einen Teil eines Quadranten eines zweidimensionalen kartesischen Koordinatensystems beinhaltet, dessen Abszisse eine Erstreckung von Parameterwerten in der Längsrichtung und dessen Ordinate eine Abweichung des Parameters von einem Sollwert angibt. Ausserdem hat die Steuereinheit eine Recheneinheit, die dazu eingerichtet ist, Dichten von Ereignissen in dem Ereignisfeld aus den Werten des Parameters und ihrer Erstreckung in der Längsrichtung zu ermitteln. Die Recheneinheit ist ausserdem dazu eingerichtet, einen Prüfgutkörper als Fläche in dem Ereignisfeld zu berechnen, welche Fläche einerseits durch die Abszisse oder eine parallel dazu verlaufende Gerade, andererseits durch die Ordinate oder eine parallel dazu verlaufende Gerade und ferner durch eine Linie in dem Ereignisfeld, welche im Wesentlichen einer konstanten Ereignisdichte folgt, begrenzt wird. Die Steuereinheit ist dazu eingerichtet, Die Fläche numerisch zu spezifizieren und mindestens einen Wert der numerischen Spezifikation als Charakteristikum des textilen Prüfgutes auszugeben.

Die erfindungsgemässe Vorrichtung kann in einer Textilverarbeitungsmaschine, bspw. einer Spinn- oder Spulmaschine für Garn, zum Einsatz kommen. Eine derartige Textilverarbeitungsmaschine weist typischerweise eine Vielzahl von Arbeitsstellen auf. Dementsprechend kann die erfindungsgemässe Vorrichtung eine Vielzahl von Messköpfen beinhalten, die sich an jeder Arbeitsstelle befinden. Die Messköpfe sind alle an der zentralen Steuereinheit angeschlossen, z. B. über einen seriellen Bus wie z. B. RS-485. Zwischen einem jeweiligen Messkopf und der Steuereinheit kann ein Schnittstellenwandler eingebaut sein. Die Steuereinheit ist vorzugsweise in der Textilverarbeitungsmaschine eingebaut.

Während die aus dem Stand der Technik bekannte Veröffentlichung WO-2010/078665 A1 durch die grafische Darstellung des Prüfgutkörpers eine intuitive Erfassung der Prüfgutcharakteristik erlaubt, charakterisiert die vorliegende Erfindung das Prüfgut exakt mit numerischen Werten. Sie tut dies aber auf ganz andere, einfachere Weise als das ebenfalls aus dem Stand der Technik bekannte System USTER® *CLASSIMAT.* Jenes System braucht zur Charakterisierung Zählwerte in 23 oder mehr Klassen; die vorliegende Erfindung kommt hingegen mit sehr wenigen - z. B. zwei bis sechs - Parametern aus und reduziert somit die für die Charakterisierung benötigte Datenmenge. Zudem ist die erfindungsgemässe Charakterisierung unter Umständen noch genauer als diejenige von USTER® *CLASSIMAT,* weil sie Werte aus einer kontinuierlichen Wertemenge liefert, nicht auf diskrete Klassen bezogene Zählwerte.

AUFZÄHLUNG DER ZEICHNUNGEN

Nachfolgend wird die Erfindung am Beispiel einer Spulmaschine für Garn anhand von Zeichnungen detailliert erläutert. Dieses Beispiel soll die Allgemeinheit nicht einschränken, denn die Erfindung lässt sich ebenso gut auf andere längliche textile Prüfgüter wie Faserband oder Vorgarn anwenden.

Figur 1      zeigt schematisch eine Spulmaschine mit einem Garnreinigersystem.

Figuren 2-7      zeigen jeweils ein Ereignisfeld mit einem oder zwei Garnkörpern und der numerischen Spezifikation gemäss dem erfindungsgemässen Verfahren.

Figur 8      zeigt Nomogramme mit Perzentilen zur Qualitätseinstufung eines erfindungsgemäss charakterisierten Garns in Bezug auf die weltweite Garnproduktion.

AUSFÜHRUNG DER ERFINDUNG

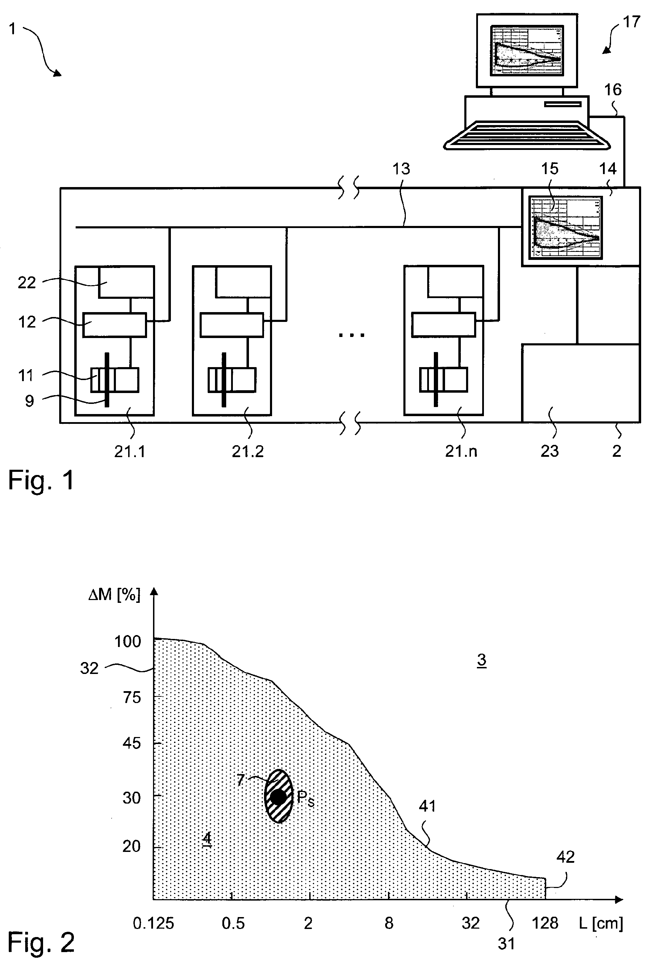

In **Figur 1** ist sehr schematisch eine Spulmaschine 2 mit mehreren Spulstellen 21.1, 21.2, ..., 21.n dargestellt. Eine erfindungsgemässe Vorrichtung 1 ist in die Spulmaschine 2 eingebaut. An jeder Spulstelle 21.1 wird während des Umspulvorgangs Garn 9 von einem Messkopf 11 der erfindungsgemässen Vorrichtung 1 überwacht. Der Messkopf 11 beinhaltet einen Sensor, mit dem eine Eigenschaft des Garns 9 gemessen wird, z. B. einen kapazitiven Sensor zur Messung einer dielektrischen Eigenschaft des Garns 9. Ferner beinhaltet der Messkopf 11 eine Auswerteeinheit, die dazu eingerichtet ist, aus den Messwerten einen Garnparameter, z. B. die Garnmasse pro Längeneinheit, zu ermitteln. Der Messkopf 11 ist über einen Schnittstellenwandler 12 mit einer zentralen Steuereinheit 14 der erfindungsgemässen Vorrichtung 1 verbunden. Über die Verbindung wird der Messkopf 11 von der Steuereinheit 14 eingestellt und gesteuert, und der Messkopf 11 übermittelt Daten wie die ermittelten Garnparameter an die Steuereinheit 14. Eine Verbindungsleitung 13 zwischen allen Schnittstellenwandlern 12 und der Steuereinheit 14 kann als serieller Bus wie z. B. RS-485 ausgebildet sein. Der Schnittstellenwandler 12 kann zusätzlich auch mit einem Spulstellenrechner 22 der jeweiligen Spulstelle 21.1 verbunden sein. Die Steuereinheit 14 weist eine Ausgabeeinheit und eine Eingabeeinheit für eine Bedienungsperson auf. Vorzugsweise sind Ausgabe- und Eingabeeinheit gemeinsam als Sensorbildschirm (Touchscreen) 15 ausgebildet. Die Steuereinheit 14 ist mit einem Steuerrechner 23 der Spulmaschine 2 verbunden. Statt in der Steuereinheit 14 können die Ausgabe- und/oder die Eingabeeinheit in der Spulmaschine 2, z. B. im Steuerrechner 23, eingebaut sein.

Die Steuereinheit 14 ist über eine Datenleitung 16 mit einer Rechnerstation 17 verbunden. Die Rechnerstation 17 ist eigenständig und vorzugsweise als Arbeitsplatzrechner (PC) mit Eingabe- und Ausgabeeinheiten ausgebildet. Sie ist für die Erfindung nicht zwingend nötig, aber vorteilhaft, um zumindest einen Teil der erfindungsgemässen Auswertungen und Ausgaben vorzunehmen und/oder die Resultate zu speichern. Sie kann vorzugsweise über ein Datennetzwerk und/oder über mobile Datenträger mit anderen Rechnerstationen Daten, insbesondere numerische Spezifikation des Garns 9, austauschen. Alternativ könnten die oben beschriebenen Aufgaben der Rechnerstation 17 von der Steuereinheit 14 übernommen werden.

**Figur 2** zeigt ein mögliches Ereignisfeld 3 mit einem Garnkörper, wie es in der Steuereinheit 14 oder der Rechnerstation 17 berechnet und auf der Ausgabeeinheit 15 dargestellt werden kann. Das Ereignisfeld 3 ist ein Quadrant eines zweidimensionalen kartesischen Koordinatensystems, das durch eine Abszisse 31 und eine Ordinate 32 aufgespannt wird. Die Ordinate 32 gibt eine Abweichung ΔM des Garnparameters, z. B. die Abweichung der Garnmasse pro Längeneinheit in Prozenten, von einem Sollwert an. Der Sollwert wird vorzugsweise durch eine laufende Mittelwertbildung über eine Vielzahl von Messungen ermittelt. Die Abszisse 31 gibt an, über welche Länge L entlang der Garnlängsrichtung sich eine jeweilige Abweichung ΔM erstreckt. Die Einteilung beider Achsen 31, 32 ist vorzugsweise logarithmisch. Eine ermittelte Abweichung ΔM und ihre Länge L bilden zusammen die Koordinaten eines Garnereignisses, das einen Punkt im Ereignisfeld 3 definiert und dort auf geeignete Weise dargestellt werden kann.

In einem Einmessvorgang wird ein genügend langer Garnabschnitt ausgemessen. Als "genügend" wird eine Einmesslänge von mindestens ca. 1 km angesehen; grössere Einmesslängen von z. B. 10 km oder 100 km Länge werden aber bevorzugt, weil sie statistisch aussagekräftigere Resultate liefern. Die Werte des Garnparameters und die dazugehörigen Längen L werden vom Messkopf 11 an die Steuereinheit 14 übermittelt. In einer Recheneinheit der Steuereinheit 14 werden daraus Dichten von Ereignissen im Ereignisfeld 3 ermittelt, so wie es z. B. in der US-6,374,152 B1 beschrieben ist. Die Ereignisdichten beziehen sich vorzugsweise auf ein kartesisches Koordinatensystem mit doppelt logarithmisch eingeteilten Achsen, wie es in den Figuren 2-7 dargestellt ist. Auf diese Weise kann jedem Punkt des Ereignisfeldes 3 eindeutig eine Ereignisdichte zugeordnet werden. Durch Interpolation, Extrapolation, Glättung und/oder andere numerische Verfahren können zu abrupte lokale Änderungen der so ermittelten Ereignisdichtefunktion, die möglicherweise durch Messfehler oder sonstige Artefakte verursacht sind, vermieden werden.

Aus der Ereignisdichtefunktion wird ein Garnkörper berechnet und als Fläche 4 im Ereignisfeld 3 dargestellt. Zu diesem Zweck wird eine genügend hohe Grenzereignisdichte von z. B. 1000 Ereignissen pro 100 km Garnlänge gewählt. Die Verbindung aller Punkte im Ereignisfeld 3, denen die Grenzereignisdichte zugeordnet ist, ergibt eine Dichtekurve 41, welche den Garnkörper vom übrigen Ereignisfeld 3 abgrenzt. Gegen die beiden Koordinatenachsen 31, 32 hin wird der Garnkörper durch die Koordinatenachsen 31, 32 selbst begrenzt. Zu grossen Längen L hin kann der Garnkörper ausserdem durch eine weitere Linie 42, die bspw. bei L = 128 cm parallel zur Ordinate 32 verläuft, begrenzt sein. Durch diese Abgrenzungen entsteht eine zusammenhängende Fläche 4, die für das ausgemessene Garn 9 charakteristisch ist. Die den Garnkörper darstellende Fläche 4 unterscheidet sich grafisch vom übrigen Ereignisfeld 3, indem sie z. B. eine andere Farbe, einen anderen Grauton und/oder ein anderes Muster aufweist als das übrige Ereignisfeld 3. Eine solche Berechnung und Darstellung des Garnkörpers werden in der WO-2010/078665 A1 ausführlich beschrieben. Sie erlauben es der Bedienungsperson, die Charakteristika des untersuchten Garns 9 schnell und intuitiv zu erfassen.

Darüber hinaus erfasst die vorliegende Erfindung die Charakteristika des untersuchten Garns 9 numerisch. Zu diesem Zweck wird die Fläche 4 numerisch spezifiziert. Im Ausführungsbeispiel von Figur 2 erfolgt die numerische Spezifikation mittels eines Schwerpunktes Ps der Fläche 4. Die Koordinaten $\underline{p}_s$ des Schwerpunktes Ps einer ebenen Fläche 4 berechnen sich bekanntlich nach der folgenden Formel:

$$\underline{p}_S = \frac{1}{A} \int_4 \underline{x} dA \quad , \tag{1}$$

worin über die ganze Fläche 4 integriert wird und

$$A = \int_4 dA \qquad\qquad (2)$$

der Flächeninhalt der Fläche 4 ist. Im Beispiel von Figur 2 hat der Flächenschwerpunkt Ps die Koordinaten $\underline{p}_s$ = (1.1 cm, 29 %). Ausserdem kann für die beiden Koordinatenwerte je ein Vertrauensintervall angegeben werden, das mit einer der gängigen statistischen Methoden bestimmt wird, bspw. 8(L) = ± 0.3 cm und $\delta(\Delta M)$ = ± 5 %. In Figur 2 ist eine elliptische Vertrauensfläche 7 um den Flächenschwerpunkt Ps herum eingezeichnet, deren Projektionen auf die jeweilige Achse den obigen Vertrauensintervallen entsprechen. Die numerische Spezifikation der Fläche 4 von Figur 2 lautet also: $\underline{p}_s$ = ((1.1 ± 0.3) cm, (29 ± 5) %).

Statt mit dem Flächenschwerpunkt Ps oder zusätzlich zu diesem kann die Fläche 4 mit der Lage ihres Konturenschwerpunktes numerisch spezifiziert werden. Der Konturenschwerpunkt kann analog zu den Formeln (1) und (2) mit Integralen definiert werden, wobei statt über die Fläche 4 über die Konturlinien, welche die Fläche 4 begrenzen, integriert wird.

Alternativ oder zusätzlich kann die Fläche 4 durch ihren Flächeninhalt A gemäss Formel (2) numerisch spezifiziert werden, wobei auch für den Flächeninhalt A ein Vertrauensintervall angegeben werden kann.

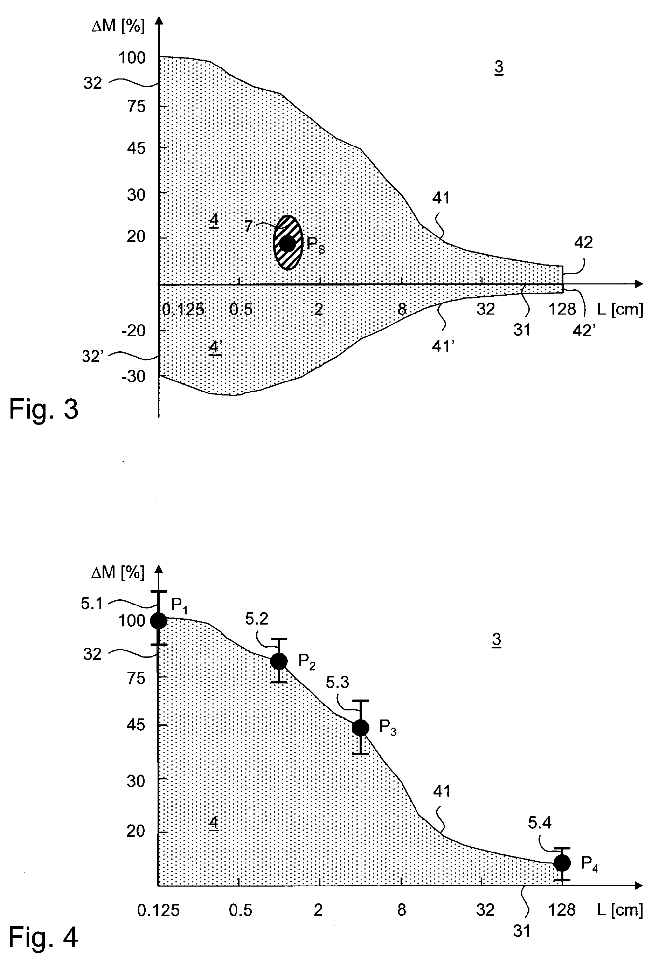

In **Figur 3** ist zusätzlich zum ersten Quadranten ein zweiter Quadrant des Ereignisfeldes 3 eingezeichnet, dessen Abszisse 31 identisch ist mit derjenigen des ersten Quadranten, dessen Ordinate 32' sich jedoch zu negativen Werten der Abweichung $\Delta M$ erstreckt. Dementsprechend sind die Ereignisse im ersten Quadranten Dickstellen im Garn 9, im zweiten Quadranten Dünnstellen. In jedem der beiden Quadranten kann eine den jeweiligen Garnkörper darstellende Fläche 4, 4' berechnet werden. Die zweite Fläche 4' im zweiten Quadranten kann auf dieselbe Weise numerisch spezifiziert werden wie die erste Fläche 4 im ersten Quadranten, bspw. mit dem Flächenschwerpunkt, dem Konturenschwerpunkte oder dem Flächeninhalt. Alternativ können die beiden Flächen 4, 4'zu einer einzigen Fläche zusammengelegt werden, und die aus der Zusammenlegung hervorgegangene einzige Fläche wird numerisch spezifiziert. Ein Flächenschwerpunkt Ps für die zusammengelegte Fläche ist in Figur 3 eingezeichnet, zusammen mit einer Vertrauensfläche 7. Die Schwerpunktskoordinaten sind in diesem Beispiel $\underline{p}_s$ = (1.1 cm, 19 %). Jedenfalls charakterisieren die beiden Flächen 4, 4'das untersuchte Garn 9 noch vollständiger als die erste Fläche 4 für Dickstellen allein.

Im Ausführungsbeispiel von **Figur 4** ist die Fläche 4 anhand der Dichtekurve 41, und näherhin durch Stützpunkte $P_1$-$P_4$, numerisch spezifiziert. Ein bestimmtes stützpunktbasiertes Spezifikationssystem wird durch die Vorgabe der Anzahl der verwendeten Stützpunkte und ihrer Lagen auf der Abszisse 31 definiert. Das Beispiel von Figur 4 hat vier Stützpunkte $P_1$-$P_4$ bei den Längen $L_1$ = 0.125 cm, $L_2$ = 1 cm, $L_3$ = 4 cm bzw. $L_4$ = 128 cm. Die y-Werte dieser Stützpunkte $P_1$-$P_4$ betragen in diesem Beispiel $\Delta M_1$ = 100 %, $\Delta M_2$ = 80 %, $\Delta M_3$ = 45 % und $\Delta M_4$ = 8 %. Sie liegen auf der Dichtekurve 41 und weisen somit im Wesentlichen dieselbe Grenzereignisdichte auf. Es ist vorteilhaft, zu jedem Stützpunkt $P_1$-$P_4$ zusätzlich ein Vertrauensintervall $\delta(\Delta M)$ für den entsprechenden $\Delta M$-Wert anzugeben. Die Vertrauensintervalle $\delta(\Delta M)$ können mit einer der gängigen statistischen Methoden bestimmt werden und sind in Figur 4 in Form von Fehlerbalken 5.1-5.4 eingezeichnet. Der Garnkörper bzw. die Fläche 4 von Figur 4 wird also mit den folgenden acht Grössen spezifiziert:

| Stützpunkt | $\Delta M$ [%] | $\delta(\Delta M)$ [%] |
|---|---|---|
| $P_1$ | 100 | ±16 |
| $P_2$ | 80 | ±12 |
| $P_3$ | 45 | ±16 |
| $P_4$ | 8 | ±8 |

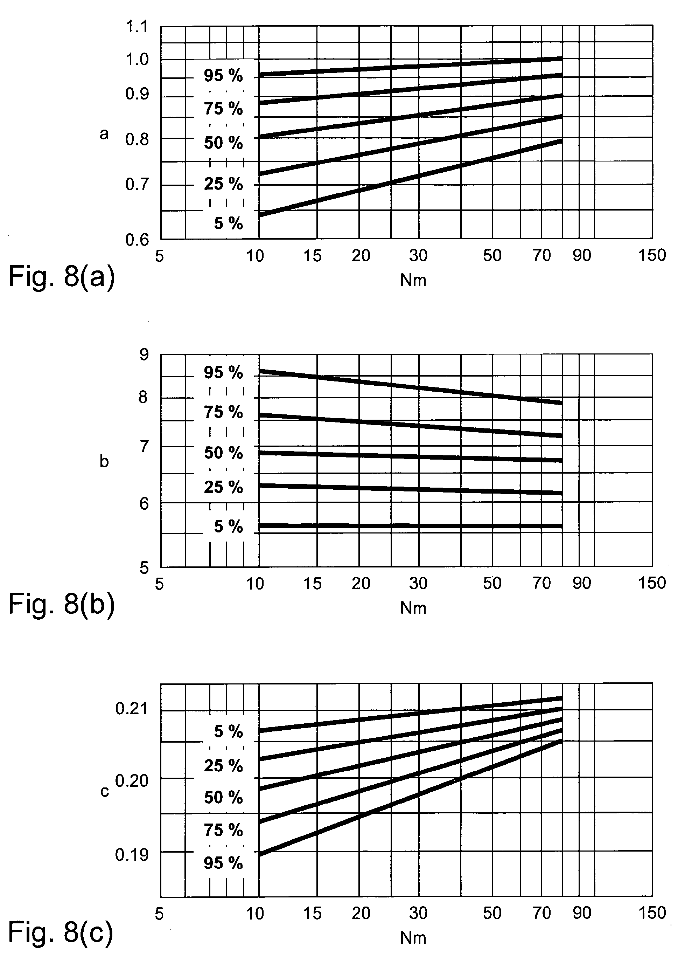

Selbstverständlich können mehr oder weniger als vier Stützpunkte verwendet werden. Die Lage der Stützpunkte wird so gewählt, dass möglichst wenige Stützpunkte das Garn 9 möglichst gut charakterisieren. Gewisse Bereiche auf der Abszisse 31 können uninteressant sein, z. B. weil in ihnen nur wenige Garnfehler vorkommen oder weil die betreffenden Garnfehler als wenig störend empfunden werden. Ob und für welche Bereiche das gilt, hängt vom jeweiligen Garntyp, von der beabsichtigten Verwendung des Garns und möglicherweise von weiteren Faktoren ab. Jedenfalls wird der Fachmann bei Kenntnis der vorliegenden Erfindung in der Lage sein, in einer gegebenen Situation möglichst wenige, möglichst charakteristische Stützpunkte anzugeben. Es ist möglich, dass sich die Fachleute auf einem bestimmten stützpunktbasierten Spezifikationssystem einigen und dieses verwenden, um untereinander Qualitätsdaten von Garnen auszutauschen. Die zu den so vorgegebenen Stützpunkten gehörigen $\Delta M$-Werte könnten dann auch in ein Qualitäts-Referenzwerk aufgenommen und in Nomogrammen in der Art von Figur 8 dargestellt werden.

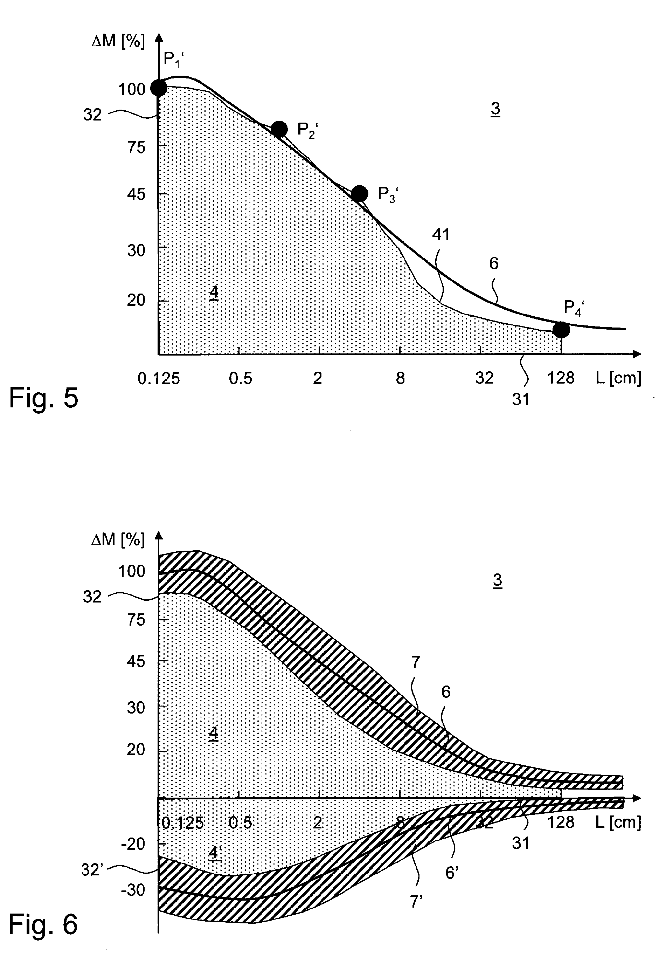

Eine andere Art der numerischen Spezifikation der Fläche 4 anhand der Dichtekurve 41 veranschaulicht **Figur 5.** Hier wird die Dichtekurve 41 durch eine Fitkurve 6 angepasst. Die Anpassung kann durch eine an sich bekannte Methode der Ausgleichsrechnung erfolgen. Vorzugsweise werden wiederum charakteristische Datenpunkte $P_1'$-$P_4'$ auf der Dichtekurve 41 gewählt, und die Datenpunkte $P_1'$-$P_4'$ werden mit einem Funktionsfit unter Berücksichtigung der Methode der kleinsten Quadrate angenähert. Ein bestimmtes fitbasiertes Spezifikationssystem wird durch die Vorgabe der Fitfunktion definiert. Nachfolgend werden beispielhaft zwei geeignete Fitfunktionen vorgestellt, denen gemeinsam ist, dass sie drei Parameter haben und für grosse L-Werte abklingen.

Ein erstes Beispiel für eine geeignete Fitfunktion ist die Funktion

$$y = a(x+b)e^{-cx} \quad , \tag{3}$$

worin x = ld(L) (Zweierlogarithmus von L), y = ld(|ΔM|) und a, b sowie c die zu findenden Funktionsparameter sind. Der Garnkörper von Figur 3 kann z. B. mit den folgenden Parameterwerten spezifiziert werden:

a = 0.82,
b = 7.6,
c = 0.20.

Möglicherweise sind nicht alle Funktionsparameter a, b, c der betreffenden Fitfunktion (3) von gleicher Bedeutung für die Charakterisierung des Garns 9. Funktionsparameter, die sich in der Praxis als wenig aussagekräftig erweisen, brauchen nicht ausgegeben zu werden, wodurch sich die Anzahl Parameterwerte, welche für die Charakterisierung des Garns 9 gebraucht werden, in vorteilhafter Weise reduziert. So könnte z. B. eine Fitfunktion mit vier Funktionsparametern verwendet werden, von denen jedoch zwei kaum mit der Garnqualität korrelieren, so dass bloss zwei Funktionsparameter das Garn 9 charakterisieren, dafür noch besser als die drei Funktionsparameter a, b, c der obigen Fitfunktion (3).

Ein zweites Beispiel für eine Fitfunktion ist die Glockenkurve

$$y = f \cdot e^{-g(x-h)^2} \quad , \tag{4}$$

worin wiederum x = ld(L), y = ld(|ΔM|) und f, g sowie h die zu findenden Funktionsparameter sind. Als beispielhafte Parameterwerte können angegeben werden:

f = 6.7,
g = 0.010.
h = -2.5.

Auch bei der fitbasierten Spezifikation ist es vorteilhaft, einen Vertrauensbereich anzugeben. Ein solcher Vertrauensbereich ist in **Figur 6** als schraffierte Fläche 7 dargestellt. Der Vertrauensbereich 7 kann z. B. ein Band sein, in welchem die Fitkurve 6 liegt. Die Breite des Vertrauensbandes 7 kann so gewählt sein, dass die "wahre" Dichtekurve mit einer gewissen Wahrscheinlichkeit, z. B. 95 %, innerhalb des Vertrauensbandes 7 liegt. Das Vertrauensband 7 kann wiederum mit numerischen Werten spezifiziert werden, z. B. durch Angabe von Vertrauensintervallen für die resultierenden Funktionsparameter, durch Angabe der Funktionen ΔM(L) für seine obere und untere Grenzlinie oder durch Angabe des Abstandes der Grenzlinien von der Fitkurve 6.

Analog zu Figur 3 ist auch in Figur 6 zusätzlich zum ersten Quadranten ein zweiter Quadrant des Ereignisfeldes 3 eingezeichnet, in den eine Fitkurve 6' für Dünnstellen und ein dazugehöriges Vertrauensband 7' gelegt ist. Kommt ein stützpunktbasiertes Spezifikationssystem (vgl. Figur 4) zum Einsatz, so können in beiden Quadranten dieselben oder unterschiedliche Stützstellen verwendet werden. Bei einem fitbasierten Spezifikationssystem (vgl. Figur 5) können für beide Quadranten dieselbe Fitfunktion oder unterschiedliche Fitfunktionen verwendet werden. Unter Verwendung der obigen Fitfunktion (3) wird der Garnkörper von Figur 6 z. B. mit den folgenden sechs (bzw. zwölf, wenn auch die Vertrauensintervalle angegeben werden) Grössen spezifiziert:

| Parameter | Dickstellen | Dünnstellen |
|-----------|-------------|-------------|
| a | 0.82 ± 0.12 | -0.99 ± 0.12 |
| b | 7.6 ± 1.5 | 5.5 ± 0.9 |
| c | 0.20 ± 0.02 | 0.20 ± 0.03 |

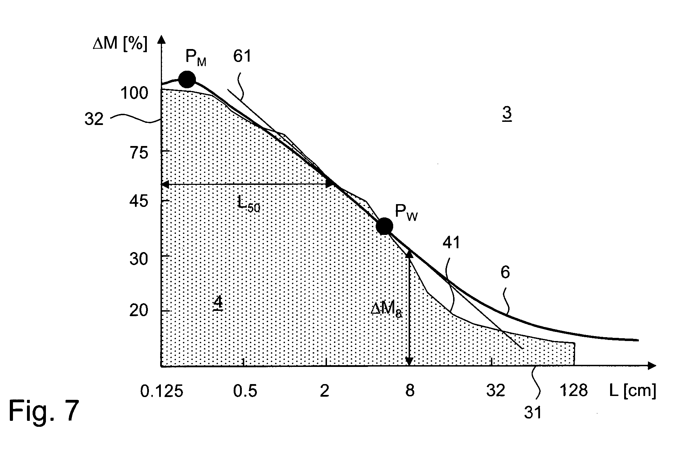

**Figur 7** zeigt weitere Möglichkeiten der numerischen Spezifizierung der Fläche 4 anhand der Dichtekurve 41. Es wird davon ausgegangen, dass die Dichtelinie 41 - wie schon in Figur 5 - durch eine Fitkurve 6 angepasst wurde. Statt der Funktionsparameter a-c der Fitfunktion (3), oder zusätzlich zu diesen, werden hier weitere numerische Werte der Fitkurve 6 berechnet und als Charakteristikum des Garns (9) ausgegeben. Solche numerischen Werte können Funktionswerte und/oder Werte der Ableitungen sein, wie sie in der mathematischen Kurvendiskussion berücksichtigt werden. Beispiele sind etwa die folgenden:

- Extrempunkte. Die Fitkurve 6 hat ein Maximum im Punkt $P_M$ mit den Koordinaten $\underline{p}_M$ = (0.18 cm, 119 %).
- Wendepunkte. Die Fitkurve 6 hat einen Wendepunkt im Punkt Pw mit den Koordinaten $\underline{p}_W$ = (5.3 cm, 34 %).
- Ein Linienschwerpunkt eines Teils der Fitkurve 6, bspw. des Teils zwischen L = 0.125 und L = 128 cm.
- Steigungen der Fitkurve 6. Die Steigungen können bei einer festen Länge L, einer festen Höhe $\Delta M$ und/oder an einem variablen Punkt wie dem Wendepunkt Pw berechnet werden. In Figur 7 ist beispielhaft eine Tangente 61 mit negativer Steigung im Wendepunkt Pw eingezeichnet.
- Punkte, in denen die Fitkurve 6 einen bestimmten Wert über- oder unterschreitet.
- Punkte, in denen die Steigung der Fitkurve 6 einen bestimmten Wert über- oder unterschreitet.
- Ein L-Wert für eine feste Höhe von bswp. $\Delta M$ = 50 %. In Figur 7 ist beispielhaft der Wert $L_{50}$ = 2.3 cm eingezeichnet. Alternativ oder zusätzlich kann der L-Wert für andere $\Delta M$ berechnet werden, die fest oder variabel sind. Ein Beispiel für den letzteren Fall ist der L-Wert bei der halben Maximumshöhe.
- Ein $\Delta M$-Wert für eine feste Länge von bspw. L = 8 cm. In Figur 7 ist beispielhaft der Wert $\Delta M_8$ = 27 %. Alternativ oder zusätzlich kann der $\Delta M$-Wert für andere Längen berechnet werden, die fest oder variabel sind. Ein Beispiel für den letzteren Fall ist der $\Delta M$-Wert bei der Hälfte der Wendepunktslänge.

Verschiedene Möglichkeiten zur numerischen Spezifikation des Garnkörpers können miteinander kombiniert werden und so gleichzeitig zur Anwendung kommen. Beispielsweise kann die Abszisse 31 in drei Bereiche aufgeteilt werden. In einem ersten Bereich kann eine erste Fitfunktion für die Dichtekurve 41, in einem zweiten Bereich können Stützpunkte und in einem dritten Bereich kann eine zweite, von der ersten Fitfunktion verschiedene Fitfunktion verwendet werden. Die oben diskutierten Möglichkeiten zur Spezifikation mittels der Dichtelinie 41 können auch bei der Spezifikation mittels der Fitkurve 6 zur Anwendung kommen, und umgekehrt.

In derselben Weise wie die Dick- und Dünnstellen können bei Bedarf weitere am Garn 9 gemessene Grössen berücksichtigt werden, z. B. ein Fremdstoffsignal.

Die numerische Spezifikation des Garnkörpers kann in ein Garnqualitäts-Referenzwerk wie die USTER® *STATISTICS* aufgenommen werden. Zu diesem Zweck wird zunächst eine für die weltweite Garnproduktion repräsentative Menge von Garnproben von Garn desselben Typs gesammelt und gemäss der vorliegenden Erfindung charakterisiert. Die numerischen Resultate der Charakterisierung werden in Nomogrammen festgehalten, wie sie in **Figur 8** dargestellt sind. Dieses Ausführungsbeispiel bezieht sich auf die obige Fitfunktion (3) mit den drei Funktionsparametern a, b, c. Die Figuren 8(a)-(c) zeigen Nomogramme, in denen jeweils einer der Parameter a, b, c gegenüber der metrischen Garnnummer Nm (Länge in Kilometern pro Kilogramm Garn) aufgetragen ist. Die Linien in den Nomogrammen geben die 5-, 25-, 50-, 75- bzw. 95-Perzentile, bezogen auf die weltweite Garnproduktion, als Funktion der Garnnummer Nm an. Das heisst: 5 % aller weltweit produzierten Garne mit einer bestimmten Garnnummer haben Parameterwerte unterhalb (bzw. im Beispiel von Fig. 8(c) oberhalb) des 5-Perzentils, 25 % aller weltweit produzierten Garne haben Parameterwerte unterhalb (bzw. im Beispiel von Fig. 8(c) oberhalb) des 25-Perzentils, etc. Das 50-Perzentil gibt den weltweiten Median an. Nomogramme wie beispielhaft in Figur 8 dargestellt erlauben eine Qualitätseinstufung eines erfindungsgemäss charakterisierten Garns 9 in Bezug auf die weltweite Garnproduktion.

Wie schon oben erwähnt, brauchen weniger aussagekräftige Funktionsparameter nicht eigens ausgegeben zu werden. Ebenfalls wurde schon oben erwähnt, dass anstelle von Funktionsparametern a, b, c die Ordinatenwerte $\Delta M_1$, $\Delta M_2$,... von Stützpunkten $P_1$, $P_2$,... in derartigen Nomogrammen dargestellt werden können. Anstatt in Nomogrammen kann die Ausgabe der numerischen Spezifikation auf andere Art erfolgen, sei es in einer anderen grafischen Darstellungsart oder in Form von numerischen Werten, die bspw. in einer Tabelle zusammengestellt sind.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören.

BEZUGSZEICHENLISTE

| | |
|---|---|
| 1 | Vorrichtung |
| 11 | Messkopf |

| 12 | Schnittstellenwandler |
|---|---|
| 13 | Verbindungsleitung |
| 14 | Steuereinheit |
| 15 | Sensorbildschirm als Eingabe- und Ausgabeeinheit |
| 16 | Datenleitung |
| 17 | Rechnerstation |
| 2 | Spulmaschine |
| 21.1, 21.2, ... | Spulstellen |
| 22 | Spulstellenrechner |
| 23 | Steuerrechner |
| 3 | Ereignisfeld |
| 31 | Abszisse |
| 32 | Ordinate |
| 4 | Garnkörper, die den Garnkörper darstellende Fläche |
| 41 | das Ereignisfeld abgrenzende Dichtekurve |
| 42 | das Ereignisfeld abgrenzende weitere Linie |
| 5.1, 5.2, ... | Fehlerbalken |
| 6 | Fitkurve |
| 61 | Tangente |
| 7 | Vertrauensfläche |
| 9 | Garn |

**Patentansprüche**

1. Verfahren zur Charakterisierung eines entlang seiner Längsrichtung bewegten länglichen textilen Prüfgutes (9), wobei

   Messwerte einer Eigenschaft des textilen Prüfgutes (9) entlang der Längsrichtung des textilen Prüfgutes (9) erfasst werden,

   Werte eines Parameters des textilen Prüfgutes aus den Messwerten ermittelt werden, ein Ereignisfeld (3) bereitgestellt wird, das einen Quadranten oder einen Teil eines Quadranten eines zweidimensionalen kartesischen Koordinatensystems beinhaltet, dessen Abszisse (31) eine Erstreckung (L) von Parameterwerten in der Längsrichtung und dessen Ordinate (32) eine Abweichung ($\Delta$M) des Parameters von einem Sollwert angibt,

   aus den Werten des Parameters und ihrer Erstreckung (L) in der Längsrichtung Dichten von Ereignissen in dem Ereignisfeld (3) ermittelt werden,

   in dem Ereignisfeld (3) ein Prüfgutkörper als Fläche (4) berechnet wird, die

   einerseits durch die Abszisse (31) oder eine parallel dazu verlaufende Gerade, andererseits durch die Ordinate (32) oder eine parallel dazu verlaufende Gerade und

   ferner durch eine Linie (41) in dem Ereignisfeld (3), welche im Wesentlichen einer konstanten Ereignisdichte folgt,

   begrenzt wird, und
   die Fläche (4) numerisch spezifiziert wird,
   **dadurch gekennzeichnet, dass**
   mindestens ein Wert (a-c) der numerischen Spezifikation als Charakteristikum des textilen Prüfgutes (9) ausgegeben wird.

2. Verfahren nach Anspruch 1, wobei die numerische Spezifikation einen Flächeninhalt der Fläche (4) berücksichtigt.

3. Verfahren nach einem der vorangehenden Ansprüche, wobei die numerische Spezifikation eine geometrische Form der Fläche (4) berücksichtigt.

4. Verfahren nach Anspruch 3, wobei die numerische Spezifikation eine Lage eines Flächenschwerpunktes und/oder eines Konturenschwerpunktes der Fläche (4) berücksichtigt.

5. Verfahren nach einem der vorangehenden Ansprüche, wobei die numerische Spezifikation einen Verlauf der Linie (41) berücksichtigt.

6. Verfahren nach Anspruch 5, wobei die numerische Spezifikation anhand von Stützpunkten ($P_1$-$P_4$) erfolgt.

7. Verfahren nach Anspruch 5, wobei die numerische Spezifikation anhand einer Ausgleichsrechnung erfolgt.

8. Verfahren nach einem der Ansprüche 5-7, wobei zur Spezifikation mindestens ein Extrempunkt ($P_M$), mindestens ein Wendepunkt (Pw), ein Linienschwerpunkt und/oder mindestens eine Kurvensteigung berechnet wird.

9. Verfahren nach einem der vorangehenden Ansprüche, wobei für die numerische Spezifikation ein Vertrauensbereich, vorzugsweise in Form von Vertrauensintervallen (5.1-5.4) oder einer Vertrauensfläche (7, 7'), ausgegeben wird.

10. Verfahren nach einem der vorangehenden Ansprüche, wobei die konstante Ereignisdichte zwischen 100 und 3000 Ereignissen pro 100 km Prüfgutlänge und vorzugsweise bei 1000 Ereignissen pro 100 km Prüfgutlänge liegt.

11. Verfahren nach einem der vorangehenden Ansprüche, wobei der Parameter im Wesentlichen einer Masse pro Längeneinheit oder einem Durchmesser des textilen Prüfgutes (9) entspricht.

12. Verfahren nach einem der vorangehenden Ansprüche, wobei in dem Ereignisfeld (3) mehrere, z. B. zwei, verschiedene Flächen (4, 4'), die vorzugsweise in verschiedenen Quadranten des Ereignisfeldes (3) liegen, numerisch spezifiziert werden.

13. Verfahren nach Anspruch 12, wobei die mehreren Flächen (4, 4') zu einer einzigen Fläche zusammengelegt und die aus der Zusammenlegung hervorgegangene einzige Fläche numerisch spezifiziert wird.

14. Verfahren nach einem der vorangehenden Ansprüche, wobei der mindestens eine Wert (a-c) der numerischen Spezifikation in ein Qualitäts-Referenzwerk für das entsprechende Prüfgut (9) aufgenommen wird.

15. Verfahren nach Anspruch 14, wobei eine für die weltweite Produktion repräsentative Menge von Proben des entsprechenden Prüfgutes (9) desselben Typs gesammelt und gemäss einem der vorangehenden Ansprüche charakterisiert wird.

16. Verfahren nach Anspruch 15, wobei im Qualitäts-Referenzwerk für den mindestens einen Wert der numerischen Spezifikation Perzentile, bezogen auf die weltweite Produktion des entsprechenden Prüfgutes (9), angegeben werden, vorzugsweise in Form eines Nomogramms.

17. Vorrichtung (1) zur Charakterisierung eines entlang seiner Längsrichtung bewegten länglichen textilen Prüfgutes (9), beinhaltend
einen Messkopf (11) zur Erfassung von Messwerten einer Eigenschaft des textilen Prüfgutes (9) entlang der Längsrichtung des textilen Prüfgutes (9) sowie zur Ermittlung von Werten eines Parameters des textilen Prüfgutes aus den Messwerten, und
eine mit dem Messkopf (11) verbundene Steuereinheit (14) mit

einer Speichereinheit und einer Ausgabeeinheit (15) zur Speicherung bzw. Ausgabe eines Ereignisfeldes (3), das einen Quadranten oder einen Teil eines Quadranten eines zweidimensionalen kartesischen Koordinatensystems beinhaltet, dessen Abszisse (31) eine Erstreckung (L) von Parameterwerten in der Längsrichtung und dessen Ordinate (32) eine Abweichung ($\Delta M$) des Parameters von einem Sollwert angibt, und
einer Recheneinheit, die dazu eingerichtet ist, Dichten von Ereignissen in dem Ereignisfeld (3) aus den Werten des Parameters und ihrer Erstreckung (L) in der Längsrichtung zu ermitteln und
einen Prüfgutkörper als Fläche (4) in dem Ereignisfeld (3) zu berechnen, welche Fläche (4)
einerseits durch die Abszisse (31) oder eine parallel dazu verlaufende Gerade,
andererseits durch die Ordinate (32) oder eine parallel dazu verlaufende Gerade und
ferner durch eine Linie (41) in dem Ereignisfeld (3), welche im Wesentlichen einer konstanten Ereignisdichte folgt,

begrenzt wird,
wobei die Steuereinheit (14) dazu eingerichtet ist, die Fläche (4) numerisch zu spezifizieren,
**dadurch gekennzeichnet,**
**dass** die Steuereinheit (14) ferner dazu eingerichtet ist, mindestens einen Wert (a-c) der numerischen Spezifikation als Charakteristikum des textilen Prüfgutes (9) auszugeben.

**18.** Textilverarbeitungsmaschine (2), bspw. Spinn- oder Spulmaschine, mit einer Vorrichtung (1) nach Anspruch 17.

**Claims**

**1.** A method for characterizing an elongated textile test material (9) which is moved along its longitudinal direction, wherein
measured values of a property of the textile test material (9) are detected along the longitudinal direction of the textile test material (9),
values of a parameter of the textile test material are determined from the measured values,
an event field (3) is provided which contains a quadrant or a part of a quadrant of a two-dimensional Cartesian coordinate system, whose abscissa (31) indicates an extension (L) of parameter values in the longitudinal direction and whose ordinate (32) indicates a deviation ($\Delta$M) of the parameter from a setpoint value,
densities of events in the event field (3) are determined from the values of the parameter and its extension (L) in the longitudinal direction,
a test material body is calculated as an area (4) in the event field (3), which is bounded

by the abscissa (31) or a straight line extending parallel thereto on the one hand, by the ordinate (32) or a straight line extending parallel thereto on the other hand, and
further by a line (41) in the event field (3) which substantially follows a constant event density, and

the area (4) is specified numerically,
**characterized in that**
at least one value (a-c) of the numerical specification is output as a characteristic of the textile test material (9).

**2.** The method according to claim 1, wherein the numerical specification considers a surface area of the area (4).

**3.** The method according to one of the preceding claims, wherein the numerical specification considers a geometrical shape of the area (4).

**4.** The method according to claim 3, wherein the numerical specification considers a position of an area centroid and/or a contour centroid of the area (4).

**5.** The method according to one of the preceding claims, wherein the numerical specification considers a shape of the line (41).

**6.** The method according to claim 5, wherein the numerical specification occurs on the basis of supporting points ($P_1$-$P_4$).

**7.** The method according to claim 5, wherein the numerical specification occurs on the basis of an adjustment calculus.

**8.** The method according to one of the claims 5 to 7, wherein for specification at least one turning point ($P_M$), at least one inflection point (Pw), a line centroid and/or at least one gradient is calculated.

**9.** The method according to one of the preceding claims, wherein a confidence range, preferably in form of confidence intervals (5.1-5.4) or a confidence area (7, 7'), is output for the numerical specification.

**10.** The method according to one of the preceding claims, wherein the constant event density lies between 100 and 3000 events per 100 km of test material length, and preferably at 1000 events per 100 km of test material length.

**11.** The method according to one of the preceding claims, wherein the parameter substantially corresponds to a mass per unit of length or a diameter of the textile test material (9).

**12.** The method according to one of the preceding claims, wherein several, e.g. two, different areas (4, 4') which preferably lie in different quadrants of the event field (3) are specified numerically.

**13.** The method according to claim 12, wherein the several areas (4, 4') are combined into one single area, and the single area produced by combination is specified numerically.

**EP 2 686 261 B1**

**14.** The method according to one of the preceding claims, wherein the at least one value (a-c) of the numerical specification is included in a quality reference document for the respective test material (9).

**15.** The method according to claim 14, wherein a set of samples of the respective test material (9) of the same type which is representative for worldwide production is collected and characterized according to one of the preceding claims.

**16.** The method according to claim 15, wherein percentiles relating to the worldwide production of the respective test material (9) are indicated in the quality reference document for the at least one value of the numerical specification, preferably in form of a nomogram.

**17.** An apparatus (1) for characterizing an elongated textile material (9) moved along its longitudinal direction, containing a measuring head (11) for detecting measured values of a property of the textile test material (9) along the longitudinal direction of the textile test material (9) and for determining values of a parameter of the textile test material from the measured values, and
a control unit (14) connected to the measuring head (11), comprising

a memory unit and an output unit (15) for storing or outputting, respectively, an event field (3) which contains a quadrant or a part of a quadrant of a two-dimensional Cartesian coordinate system, whose abscissca (31) indicates an extension (L) of parameter values in the longitudinal direction and whose ordinate (32) indicates a deviation ($\Delta$M) of the parameter from a setpoint value, and
a computing unit which is configured

to determine densities of events in the event field (3) from the values of the parameter and its extension (L) in the longitudinal direction, and
to calculate a test material body as an area (4) in the event field (3), which area (4) is bounded

by the abscissa (31) or a line extending parallel thereto on the one hand,
by the ordinate (32) or a line extending parallel thereto on the other hand, and
further by a line (41) in the event field (3) which substantially follows a constant event density,

wherein the control unit (14) is configured to specify the area (4) numerically, **characterized in that**
the control unit (14) is configured to output at least one value (a-c) of the numerical specification as a characteristic of the textile test material (9).

**18.** A textile processing machine (2), e.g., a spinning or winding machine, with an apparatus (1) according to claim 17.

**Revendications**

**1.** Procédé pour caractériser un produit textile à contrôler allongé (9) déplacé dans le sens de sa longueur, dans lequel des valeurs de mesure d'une propriété du produit textile à contrôler (9) sont acquises dans le sens de la longueur du produit textile à contrôler (9) ;
des valeurs d'un paramètre du produit textile à contrôler sont déterminées à partir des valeurs de mesure ;
un champ d'événements (3) contenant un quadrant ou une partie de quadrant d'un système de coordonnées cartésiennes à deux dimensions, dont l'abscisse (31) indique une étendue (L) de valeurs de paramètres dans le sens de la longueur et dont l'ordonnée (32) indique un écart ($\Delta$M) du paramètre par rapport à une valeur de consigne, est créé ;
des densités d'événements dans le champ d'événements (3) sont déterminées à partir des valeurs du paramètre et de leur étendue (L) dans le sens de la longueur ;
un corps d'article à contrôler est calculé dans le champ d'événements (3) sous la forme d'une aire (4) délimitée

d'une part par l'abscisse (31) ou une droite parallèle à celle-ci,
d'autre part par l'ordonnée (32) ou une droite parallèle à celle-ci, et
en outre par une ligne (41) dans le champ d'événements (3) qui suit pour l'essentiel une densité d'événements constante ; et

l'aire (4) est spécifiée de façon numérique,

**EP 2 686 261 B1**

**caractérisé en ce**
qu'au moins une valeur (a-c) de la spécification numérique est émise en sortie comme caractéristique du produit textile à contrôler (9).

2. Procédé selon la revendication 1, dans lequel la spécification numérique tient compte d'un contenu de l'aire (4).

3. Procédé selon l'une des revendications précédentes, dans lequel la spécification numérique tient compte d'une forme géométrique de l'aire (4).

4. Procédé selon la revendication 3, dans lequel la spécification numérique tient compte d'une position d'un barycentre de l'aire et/ou d'un barycentre de contour de l'aire (4).

5. Procédé selon l'une des revendications précédentes, dans lequel la spécification numérique tient compte d'un tracé de la ligne (41).

6. Procédé selon la revendication 5, dans lequel la spécification numérique est établie à l'aide de points d'appui ($P_1$-$P_4$).

7. Procédé selon la revendication 5, dans lequel la spécification numérique est établie à l'aide d'un calcul de compensation.

8. Procédé selon l'une des revendications 5 à 7, dans lequel, en vue de la spécification, au moins un point extrême ($P_M$), au moins un point d'inflexion (Pw), un barycentre de ligne et/ou au moins une pente de courbe sont calculés.

9. Procédé selon l'une des revendications précédentes, dans lequel, en vue de la spécification numérique, un intervalle de confiance est émis en sortie, de préférence sous la forme d'intervalles de confiance (5.1-5.4) ou d'une aire de confiance (7, 7').

10. Procédé selon l'une des revendications précédentes, dans lequel la densité d'événements constante est comprise entre 100 et 3000 événements par 100 km de longueur de produit textile à contrôler et, de préférence, est de 1000 événements par 100 km de longueur de produit textile à contrôler.

11. Procédé selon l'une des revendications précédentes, dans lequel le paramètre correspond pour l'essentiel à une masse par unité de longueur ou à un diamètre du produit textile à contrôler (9).

12. Procédé selon l'une des revendications précédentes, dans lequel plusieurs, par exemple deux, aires différentes (4, 4'), qui se trouvent de préférence dans des quadrants différents du champ d'événements (3), sont spécifiées de façon numérique dans le champ d'événements (3).

13. Procédé selon la revendication 12, dans lequel les plusieurs aires (4, 4') sont réunies en une aire unique et l'aire unique résultant de la réunion est spécifiée de façon numérique.

14. Procédé selon l'une des revendications précédentes, dans lequel l'au moins une valeur (a-c) de la spécification numérique est incluse dans un référentiel de qualité pour le produit à contrôler (9) correspondant.

15. Procédé selon la revendication 14, dans lequel une quantité d'échantillons du produit à contrôler (9) du même type représentative de la production mondiale est collectée et caractérisée selon l'une des revendications précédentes.

16. Procédé selon la revendication 15, dans lequel le référentiel de qualité indique pour l'au moins une valeur de la spécification numérique des percentiles par rapport à la production mondiale du produit à contrôler (9) correspondant, de préférence sous la forme d'un nomogramme.

17. Dispositif (1) pour la caractérisation d'un produit textile à contrôler allongé (9) déplacé dans le sens de sa longueur, comprenant :

une tête de mesure (11) destinée à acquérir des valeurs de mesure d'une propriété du produit textile à contrôler (9) dans le sens de la longueur du produit textile à contrôler (9) et à déterminer des valeurs d'un paramètre du produit textile à contrôler à partir des valeurs de mesure, et
une unité de commande (14) reliée à la tête de mesure (11), avec

une unité de mémoire et une unité de sortie (15) pour enregistrer et émettre en sortie un champ d'événements (3) contenant un quadrant ou une partie de quadrant d'un système de coordonnées cartésiennes à deux dimensions, dont l'abscisse (31) indique une étendue (L) de valeurs de paramètres dans le sens de la longueur et dont l'ordonnée (32) indique un écart ($\Delta$M) du paramètre par rapport à une valeur de consigne et une unité de calcul arrangée pour

déterminer des densités d'événements dans le champ d'événements (3) à partir des valeurs du paramètre et de leur étendue (L) dans le sens de la longueur et calculer un corps de produit à contrôler sous la forme d'une aire (4) dans le champ d'événements (3), laquelle aire (4) est délimitée

d'une part par l'abscisse (31) ou une droite parallèle à celle-ci,

d'autre part par l'ordonnée (32) ou une droite parallèle à celle-ci et

en outre par une ligne (41) dans le champ d'événements (3) qui suit pour l'essentiel une densité d'événements constante,

l'unité de commande (14) étant conçue pour spécifier l'aire (4) de façon numérique,
**caractérisé en ce que**
l'unité de commande (14) est en outre conçue pour émettre en sortie au moins une valeur (a-c) de la spécification numérique comme caractéristique du produit textile à contrôler (9).

18. Machine textile (2), par exemple machine à filer ou bobineuse, avec un dispositif (1) selon la revendication 17.

**Fig. 1**

**Fig. 2**

Fig. 3

Fig. 4

Fig. 5

Fig. 6

Fig. 7

Fig. 8(a)

Fig. 8(b)

Fig. 8(c)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0439767 A2
- EP 0924513 A1
- WO 2004044579 A1
- US 5537811 A
- WO 2010078665 A1
- US 6374152 B1

**In der Beschreibung aufgeführte Nicht-Patentliteratur**

- USTER® QUANTUM 2 - Online-Qualitätsmanagement in der Spulerei. Uster Technologies AG, Mai 2005
- USTER® CLASSIMAT QUANTUM. Uster Technologies AG, August 2007